# EUROPÄISCHE PATENTSCHRIFT

(11) **EP 3 063 121 B1**
(45) Veröffentlichungstag und Bekanntmachung des Hinweises auf die Patenterteilung: **13.12.2017**
(21) Anmeldenummer: 14792386.6
(22) Anmeldetag: 09.10.2014
(51) Int. Cl.: C07C 67/08, C07C 67/54, C07C 67/58, C07C 67/60

(54) **VERFAHREN ZUR GEWINNUNG VON MIT POLYOLESTERN ANGEREICHERTEN PRODUKTSTRÖMEN AUS DEN NEBENSTRÖMEN DER POLYOLESTERHERSTELLUNG**
METHOD FOR OBTAINING POLYOL ESTERS-ENRICHED PRODUCT STREAMS FROM THE SIDE-STREAMS IN POLYOL ESTER PRODUCTION
PROCÉDÉ D'OBTENTION DE COURANTS DE PRODUIT ENRICHIS AVEC DES ESTERS DE POLYOL À PARTIR DES COURANTS SECONDAIRES DE PRODUCTION D'ESTER DE POLYOL

(30) Priorität: 02.11.2013 DE 102013018456
(43) Veröffentlichungstag der Anmeldung: 07.09.2016
(73) Patentinhaber: OXEA GmbH, 46147 Oberhausen (DE)
(72) Erfinder: KUBITSCHKE, Jens, 45127 Essen (DE); KREICKMANN, Thorsten, 45239 Essen (DE); ARNOLD, Jörg, 46535 Dinslaken (DE); STRUTZ, Heinz, 47445 Moers (DE)
(86) Internationale Anmeldenummer: PCT/EP2014/002737
(87) Internationale Veröffentlichungsnummer: WO 2015/062700

(56) Entgegenhaltungen:
- DE-A1-102009 048 772
- DE-A1-102009 048 773

## Beschreibung

Die Erfindung betrifft ein Verfahren zur Gewinnung von mit Polyolestern angereicherten Produktströmen aus den Nebenströmen der Polyolesterherstellung.

Ester mehrwertiger Alkohole, auch Polyolester genannt, finden in großem Umfang und in vielfältiger Weise Anwendung in der Technik, wie zum Beispiel als Weichmacher oder Schmiermittel. Durch die Auswahl geeigneter Ausgangsprodukte lassen sich die physikalischen Stoffeigenschaften, wie zum Beispiel Siedepunkt oder Viskosität, gezielt einstellen und den chemischen Eigenschaften, wie der Hydrolyseresistenz oder der Stabilität gegenüber einem oxidativen Abbau, Rechnung tragen. Auch auf die Lösung konkreter anwendungstechnischer Probleme können Polyolester gezielt zugeschnitten werden. Ausführliche Übersichten über den Einsatz von Polyolestern finden sich zum Beispiel in Ullmann's Encyclopedia of Industrial Chemistry, 5. Auflage, 1985, VCH Verlagsgesellschaft, Vol. A1, Seiten 305-319; 1990, Vol. A15, Seiten 438-440 oder in Kirk Othmer, Encyclopedia of Chemical Technology, 3. Auflage, John Wiley & Sons, 1978, Vol. 1, Seiten 778-787; 1981, Vol. 14, Seiten 496-498.

Die Verwendung von Polyolestern als Schmierstoffe besitzt eine große technische Bedeutung und sie werden besonders für solche Anwendungsgebiete eingesetzt, in denen Schmierstoffe auf Mineralölbasis die gesetzten Anforderungen nur unvollständig erfüllen. Polyolester werden insbesondere als Turbinenmotoren- und Instrumentenöle benutzt. Polyolester für Schmiermittelanwendungen basieren häufig auf 1,3-Propandiol, 1,3-Butandiol, 1,4-Butandiol, 1,2-Hexandiol, 1,6-Hexandiol, Neopentylglykol, Trimethylolpropan, Pentaerythrit, 2,2,4-Trimethylpentan-1,3-diol, Glycerin oder 3(4),8(9)-Di-hydroxymethyltricyclo[5.2.1.0^{2.6}]decan, auch als TCD-Alkohol DM bezeichnet, als Alkoholkomponente.

Auch als Weichmacher werden Polyolester in erheblichem Umfang verwendet. Weichmacher finden in vielfältiger Weise Anwendung in Kunststoffen, Beschichtungsmitteln, Dichtungsmassen, Kautschuk- und Gummiartikeln. Sie treten mit hochpolymeren thermoplastischen Stoffen, ohne chemisch zu reagieren, vorzugsweise durch ihre Löse- und Quellvermögen in physikalische Wechselwirkung. Hierdurch bildet sich ein homogenes System aus, dessen thermoplastischer Bereich gegenüber den ursprünglichen Polymeren zu niederen Temperaturen verschoben ist, u. a. mit dem Ergebnis, dass seine mechanischen Eigenschaften optimiert, z. B. Formveränderungsvermögen, Elastizität, Festigkeit erhöht werden und die Härte verringert wird.

Eine spezielle Klasse von Polyolestern (sie werden kurz als G-Ester bezeichnet) enthält als Alkoholkomponente Diole bzw. Etherdiole, beispielsweise Ethylenglykol, Diethylenglykol, Triethylenglykol, Tetraethylenglykol, 1,2-Propylenglykol oder höhere Propylenglykole. Ihre Herstellung kann auf unterschiedlichen Wegen erfolgen. Neben der Umsetzung von Alkohol und Säure gegebenenfalls in Gegenwart saurer Katalysatoren werden in der Praxis weitere Prozesse zur Gewinnung von G-Estern angewandt, u. a. die Reaktion von Diol mit Säurehalogenid, die Umesterung eines Carbonsäureesters mit einem Diol und die Addition von Ethylenoxid an Carbonsäuren (Ethoxylierung). In der industriellen Fertigung haben sich lediglich die unmittelbare Umsetzung von Diol und Carbonsäure und die Ethoxylierung von Carbonsäuren als Produktionsverfahren durchgesetzt, wobei der Veresterung von Diol und Säure zumeist der Vorzug gegeben wird. Denn dieses Verfahren kann ohne besonderen Aufwand in konventionellen chemischen Apparaten durchgeführt werden und es liefert chemisch einheitliche Produkte. Demgegenüber erfordert die Ethoxylierung umfangreiche und kostenintensive technische Mittel.

Die unmittelbare Veresterung von Alkoholen mit Carbonsäuren gehört zu den Grundoperationen der organischen Chemie. Um die Reaktionsgeschwindigkeit zu erhöhen, führt man die Umsetzung üblicherweise in Gegenwart von Katalysatoren durch. Der Einsatz eines der Reaktanten im Überschuss und/oder die Abtrennung des im Verlauf der Reaktion gebildeten Wassers stellt sicher, dass das Gleichgewicht entsprechend dem Massenwirkungsgesetz zur Seite des Reaktionsproduktes, also des Esters, verschoben wird, d. h. hohe Ausbeuten erzielt werden.

Umfassende Angaben zur Herstellung von Estern mehrwertiger Alkohole, darunter auch Estern aus Ethylenglykolen und Fettsäuren und zu den Eigenschaften ausgewählter Vertreter dieser Verbindungsklassen finden sich in Goldsmith, Polyhydric Alcohol Esters of Fatty Acids, Chem. Rev. 33, 257 ff. (1943). Beispielsweise erfolgt die Herstellung von Estern des Diethylenglykols, des Triethylenglykols und von Polyethylenglykolen über Reaktionszeiten von 2,5 bis 8 Stunden bei Temperaturen von 130 bis 230°C. Als geeignete Katalysatoren für die Veresterung mehrwertiger Alkohole werden anorganische Säuren, saure Salze, organische Sulfonsäuren, Acetylchlorid, Metalle oder amphotere Metalloxide genannt. Die Entfernung des Reaktionswassers erfolgt mit Hilfe eines Schleppmittels, beispielsweise Toluol oder Xylol oder durch Einleiten inerter Gase wie Kohlendioxid oder Stickstoff.

Auf die Gewinnung und die Eigenschaften von Fettsäureestern der Polyethylenglykole geht Johnson (Edit.), Fatty Acids in Industry (1989) Kap. 9, Polyoxyethylene Esters of Fatty Acid ein und gibt eine Reihe präparativer Hinweise. Höhere Diesterkonzentrationen erzielt man durch die Erhöhung des molaren Verhältnisses von Carbonsäure zu Glykol. Geeignete Maßnahmen zur Entfernung des Reaktionswassers sind die Azeotropdestillation in Gegenwart eines mit Wasser nicht mischbaren Lösungsmittels, das Erhitzen unter Durchleiten eines Inertgases oder die Durchführung der Reaktion unter Vakuum in Gegenwart eines Trockenmittels. Verzichtet man auf den Zusatz von Katalysatoren, so sind längere Reaktionszeiten und höhere Reaktionstemperaturen erforderlich. Beide Reaktionsbedingungen können durch den Einsatz von Katalysatoren gemildert werden. Neben Schwefelsäure sind organische Säuren wie p-Toluolsulfonsäure sowie Kationenaustauscher vom Polystyroltyp die bevorzugten Katalysatoren. Auch die Verwendung von Metallpulvern, wie Zinn oder Eisen wird beschrieben. Nach der Lehre von US 2,628,249 lassen sich Farbprobleme bei der Katalyse mit Schwefelsäure oder Sulfonsäure mildern, wenn in Gegenwart von Aktivkohle gearbeitet wird.

Als weitere metallhaltige Katalysatoren werden auch Titan-, Zirconium- oder Zinnalkoholate, -carboxylate oder -chelate zur Herstellung von Polyolestern, beispielsweise gemäß US 5,324,853 A1, eingesetzt. Solche Metallkatalysatoren kann man als Hochtemperaturkatalysatoren ansehen, denn sie erreichen ihre volle Aktivität erst bei hohen Veresterungstemperaturen, im Allgemeinen oberhalb von 180°C. Sie werden häufig nicht zu Beginn der Veresterungsreaktion zugesetzt sondern nachdem das Reaktionsgemisch bereits aufgeheizt wurde und zum Teil unter Wasserabspaltung reagiert hat. Trotz der im Vergleich zur klassischen Schwefelsäurekatalyse erforderlichen höheren Reaktionstemperaturen und längeren Reaktionszeiten werden bei der Katalyse mit solchen metallhaltigen Verbindungen Rohester mit einer vergleichsweise geringen Farbzahl erhalten. Gängige Veresterungskatalysatoren sind beispielsweise Tetra(isopropyl)orthotitanat, Tetra(butyl)orthotitanat, Tetra(butyl)zirconat oder Zinn(II)-2-ethylhexanoat. Weitere Verfahren zur Herstellung von Polyolestern werden beispielsweise in DE 10 2009 048 771 A1, DE 10 2009 048 772 A1 und DE 10 2009 048 775 A1 behandelt. Nach diesen Verfahren wird der erhaltene Rohester im Zuge der Aufarbeitung einer Wasserdampfbehandlung unterzogen.

Es ist ebenfalls bekannt, während des Aufarbeitungsverfahrens des nach der Veresterungsstufe erhaltenen Rohesters eine Behandlung mit einer peroxidischen Verbindung vorzunehmen, um die Farbzahl des Polyolesters zu verbessern (DE 10 2009 048 773 A1). Ein analoges Verfahren unter Verwendung von Ozon oder ozonhaltigen Gasen zur Farbaufhellung von Polyolestern wird in DE 10 2009 048 774 A1 beschrieben. Beiden Verfahren ist gemeinsam, dass nach der oxidativen Behandlung sich unmittelbar ohne weitere Zwischenschritte eine Wasserdampfbehandlung anschließt. Vorteilhafterweise werden im Verlauf der Wasserdampfbehandlung überschüssige peroxidische oder ozonhaltige Verbindungen zerstört und eingetragenes Wasser entfernt.

Der bei der Wasserdampfbehandlung des Rohesters abgeführte Strom enthält jedoch substantielle Mengen an dem gewünschten Polyolester zusammen mit einer Reihe weiterer Nebenkomponenten. Im Allgemeinen enthält der im Zuge der Wasserdampfbehandlung abgeführte Stoffstrom, den man auch als Nebenstrom ansehen kann, bezogen auf den organischen Anteil 1 - 30 Gew.-% Monoester, 40 - 80 Gew.-% Polyolester sowie als Rest auf 100 Gew.-% Nebenkomponenten wie Ausgangscarbonsäure und deren Ester, Leichtsieder und Hochsieder.

Da der Gehalt an Polyolester in dem mit Wasserdampf abgeführten Nebenstrom vergleichsweise hoch ist, besteht Bedarf an einem Verfahren, aus diesem Nebenstrom der Polyolesterherstellung einen mit Polyolestern angereicherten Produktstrom zurückzugewinnen und in den Prozess der Polyolesterherstellung zurückzuführen. Durch die Gewinnung dieser zusätzlichen Polyolestermengen wird die Rohstoffeffizienz der Gesamtreaktion verbessert und die Kapazität der Produktionsanlage bei gleicher Anlagenkonfiguration ohne aufwendige Investitionen deutlich erhöht.

Die Erfindung besteht daher in einem Verfahren zur Gewinnung von mit Polyolestern angereicherten Produktströmen aus den Nebenströmen der Polyolesterherstellung, umfassend
a) die Umsetzung von Polyolen der allgemeinen Formel (II)

   H-(-O-[-CR¹R²-]ₘ-)ₒ-OH (II)

   in der R¹ und R² unabhängig voneinander Wasserstoff, einen Alkylrest mit 1 bis 5 Kohlenstoffatomen, vorzugsweise Methyl, Ethyl oder Propyl, oder einen Hydroxyalkylrest mit 1 bis 5 Kohlenstoffatomen, vorzugsweise der Hydroxymethylrest, m eine ganze Zahl von 1 bis 10, vorzugsweise 1 bis 8 und insbesondere 1, 2, 3 oder 4, o eine ganze Zahl von 2 bis 15, vorzugsweise 2 bis 8 und insbesondere 2, 3, 4 oder 5 bedeuten, mit linearen oder verzweigten aliphatischen Monocarbonsäuren mit 3 bis 20 Kohlenstoffatomen ausgewählt aus der Gruppe von Propionsäure, n-Buttersäure, Isobuttersäure, n-Pentansäure, 2-Methylbuttersäure, 3-Methylbuttersäure, 2-Methylpentansäure, n-Hexansäure, 2-Ethylbuttersäure, n-Heptansäure, 2-Methylhexansäure, 2-Ethylhexansäure, n-Nonansäure, 2-Methyloctansäure, Isononansäure, 3,5,5-Trimethylhexansäure oder 2-Propylheptansäure unter Entfernung des gebildeten Wassers;
b) die Abtrennung unumgesetzter Ausgangsverbindungen von dem gebildeten Rohester;
c) die Behandlung des gemäß Schritt b) erhaltenen Rohesters mit Wasserdampf unter Bildung eines flüchtigen Nebenstromes; und
d) die Abtrennung des in Schritt c) erhaltenen flüchtigen Nebenstromes, dadurch gekennzeichnet, dass der in Schritt d) erhaltene flüchtige Nebenstrom in eine wässrige Phase und eine organische Phase getrennt wird und die abgetrennte organische Phase einer weiteren Behandlung mit Wasserdampf unterzogen wird und ein mit Polyolestern angereicherter Produktstrom als Rückstand gewonnen wird.

Überraschenderweise wurde gefunden, dass der flüchtige Nebenstrom, der nach der Behandlung des Rohesters mit Wasserdampf anfällt und abgetrennt wird, einer weiteren Wasserdampfbehandlung unterzogen werden kann, in deren Verlauf sich der Gehalt an dem gewünschten Polyolester aufkonzentrieren lässt.

Der flüchtige Nebenstrom aus der Rohesterbehandlung wird abgetrennt und in ein Kondensationsgefäß geleitet, in dem dieser Nebenstrom sich in eine wässrige Phase und in eine organische Phase trennt. Die organische Phase enthält neben dem gewünschten Polyolester auch noch die Ausgangsverbindungen Polyol und Monocarbonsäure, Monoester sowie Abbauprodukte, insbesondere bei der Veresterung von Etherdiolen. Im Allgemeinen beträgt der Polyolestergehalt 40 bis 80 Gew.-% in der abgetrennten organischen Phase aus dem flüchtigen Nebenstrom.

Zur Aufkonzentrierung des Polyolestergehaltes wird die aus dem wasserhaltigen Nebenstrom abgetrennte organische Phase erneut einer weiteren Wasserdampfbehandlung unterzogen, die beispielsweise in einfacher Form durch Einleiten von Wasserdampf erfolgen kann. Hierbei werden leichter siedende Verbindungen, wie die Ausgangsverbindungen Polyol oder Monocarbonsäure, sowie Monoester entfernt. Die Bedingungen dieser weiteren Wasserdampfbehandlung sind gezielt einzustellen, um auf der einen Seite eine ausreichende Abtrennung der flüchtigen Bestandteile zu erzielen und auf der anderen Seite einen zu starken Anstieg der Farbzahl durch thermische Belastung zu vermeiden. Die weitere Wasserdampfbehandlung wird im Allgemeinen bei Normaldruck durchgeführt, obwohl die Anwendung eines leichten Unterdrucks beispielsweise bis 400 hPa nicht ausgeschlossen ist. Man arbeitet bei Temperaturen in einem Bereich von 120 bis 260°C, vorzugsweise von 150 bis 220°C, bis zu einem akzeptablen Polyolestergehalt, im Allgemeinen über einen Zeitraum von 1 bis 10 Stunden, vorzugsweise von 1 bis 5 Stunden. Zweckmäßigerweise wendet man ein stufenförmiges Temperaturprofil an, beispielsweise wird mit der Wasserdampfbehandlung bei 120°C begonnen und die Behandlungstemperatur ein-, zwei- oder mehrstufig von Stufe zu Stufe erhöht. Gegebenenfalls kann gleichzeitig der Druck ausgehend von Normaldruck stufenweise reduziert werden. Die einzustellenden Temperatur- und Druckbedingungen auf den jeweiligen Stufen, die Zahl der Stufen sowie die jeweilige Temperaturerhöhungs- oder Druckminderungsrate pro Zeiteinheit können über einen weiten Bereich variiert werden und richten sich nach den Vorgaben für den Restgehalt an flüchtigen Komponenten und den Spezifikationswerten für die Farbzahl. Je intensiver die Wasserdampfbehandlung betrieben wird, desto stärker lässt sich der Restgehalt an flüchtigen Komponenten zwar reduzieren aber desto größer ist die Gefahr der Farbzahlverschlechterung. Daher sind die Bedingungen für die weitere Wasserdampfbehandlung gezielt einzustellen, um einen vertretbaren Kompromiss zwischen dem Gehalt an dem gewünschten Polyolester in dem behandelten Nebenstrom und der Farbzahl zu erreichen.

Beispielsweise wird bei Normaldruck ausgehend von 150°C zunächst über eine Stunde die weitere Wasserdampfbehandlung durchgeführt, anschließend die Temperatur auf 180°C angezogen und bei dieser Temperatur über eine weitere Stunde behandelt und anschließend nach Temperaturerhöhung auf 200°C die Wasserdampfbehandlung über einen weiteren Zeitraum von zwei Stunden zu Ende geführt.

In einer Ausgestaltung des erfindungsgemäßen Verfahrens kann die Wasserdampfbehandlung in Gegenwart eines Adsorbens durchgeführt werden. Man verwendet dabei poröse, großflächige feste Materialien, die üblicherweise in der chemischen Praxis sowohl im Labor als auch in technischen Anlagen eingesetzt werden. Beispiele für solche Materialien sind oberflächenreiche Polykieselsäuren wie Silicagele (Kiesel-Xerogele), Kieselgel, Kieselgur, oberflächenreiche Aluminiumoxide und Aluminiumoxidhydrate, mineralische Materialien, wie Tone oder Carbonate, oder Aktivkohle. Besonders bewährt hat sich Aktivkohle. Im Allgemeinen ist das Adsorbens feinteilig in der abgetrennten organischen Phase suspendiert, die durch intensives Rühren und durch Einleiten von Wasserdampf bewegt wird. Dadurch wird ein inniger Kontakt zwischen der flüssigen Phase und dem Adsorbens erreicht. Die Menge des Adsorbens kann weitgehend frei und somit den individuellen Erfordernissen entsprechend eingestellt werden. Bezogen auf 100 Gewichtsteile der flüssigen Phase bewährt es sich 0,1 bis 5, vorzugsweise 0,5 bis 1,5 Gewichtsteile des Adsorbens einzusetzen. Anschließend wird das Adsorbens in konventionellen Filtrierapparaturen bei normaler Temperatur oder bei Temperaturen bis 120°C, gegebenenfalls in Gegenwart gängiger Filtrierhilfsmittel wie Cellulose, Kieselgel, Kieselgur oder Holzmehl abfiltriert.

Der nachbehandelte Polyolester fällt als flüssiger Rückstand bei der weiteren Wasserdampfbehandlung an und enthält im Allgemeinen Wertprodukt mit einem Gehalt von mehr als 80 Gew.-%, vorzugsweise mehr als 90 Gew.-%, jeweils bezogen auf den flüssigen Rückstand. Anschließend wird der so zurückgewonnene Polyolester in den Produktionsprozess zurückgeführt.

Falls erforderlich, kann die Farbzahl des zurückgewonnenen Polyolesters durch Behandlung mit einer oxidierenden Verbindung und unmittelbar anschließender Wasserdampfbehandlung erniedrigt werden, beispielsweise durch Behandlung mit einer wässrigen Wasserstoffperoxidlösung, wie in DE 10 2009 048 773 A1 beschrieben, oder durch Behandlung mit Ozon oder ozonhaltigen Gasen gemäß dem aus DE 10 2009 048 774 A1 bekannten Verfahren.

Die Reaktion zwischen den Ausgangsverbindungen Polyol und der aliphatischen Monocarbonsäure setzt in Abhängigkeit von den Einsatzmaterialien im Bereich von etwa 120 bis 180°C ein und kann anschließend auf unterschiedlich ausgestaltete Weise zu Ende geführt werden.

Nach einer Ausgestaltung des erfindungsgemäßen Verfahrens wird zunächst ausgehend von Raumtemperatur auf eine Temperatur bis auf maximal 280°C, vorzugsweise bis auf 250°C erhitzt und bei konstant gehaltener Temperatur der Druck ausgehend von Normaldruck stufenweise erniedrigt, um die Entfernung des Reaktionswassers zu erleichtern. Die Wahl der Druckstufen, ob ein-, zwei- oder mehrstufig, sowie der auf der jeweiligen Stufe einzustellende Druck kann über einen weiten Bereich variiert und den jeweiligen Bedingungen angepasst werden. Beispielsweise kann in einer ersten Stufe der Druck ausgehend von Normaldruck zunächst bis auf 600 hPa erniedrigt werden und anschließend die Reaktion bei einem Druck von 300 hPa zu Ende geführt werden. Bei diesen Druckangaben handelt es sich um Richtwerte, die zweckmäßig eingehalten werden.

Neben der Variation des Drucks kann ebenfalls auch die Temperatur ausgehend von Raumtemperatur während der Veresterungsreaktion ein-, zwei- oder mehrstufig verändert werden, so dass bei konstant eingestelltem Druck die Temperatur von Stufe zu Stufe erhöht wird, üblicherweise bis auf eine maximale Temperatur von 280°C. Es hat sich aber als zweckmäßig erwiesen, bei von Stufe zu Stufe ansteigender Temperatur auf maximal 280°C zu erhitzen und auch den Druck von Stufe zu Stufe zu erniedrigen. Beispielsweise kann die Veresterungsreaktion ausgehend von Raumtemperatur in einer ersten Stufe bei einer Temperatur bis auf 190°C geführt werden. Ebenfalls wird ein verminderter Druck bis auf 600 hPa angelegt, um das Austreiben des Reaktionswassers zu beschleunigen. Nach Erreichen der Temperaturstufe von 190°C wird der Druck nochmals bis auf 300 hPa erniedrigt und die Veresterungsreaktion bei einer Temperatur bis auf 230°C zu Ende geführt. Bei diesen Temperatur- und Druckangaben handelt es sich um Richtwerte, die zweckmäßiger Weise eingehalten werden. Die einzustellenden Temperatur- und Druckbedingungen auf den jeweiligen Stufen, die Zahl der Stufen sowie die jeweilige Temperaturerhöhungs- oder Druckminderungsrate pro Zeiteinheit können über einen weiten Bereich variiert werden und entsprechend den physikalischen Eigenschaften der Ausgangsverbindungen und der Reaktionsprodukte angepasst werden, wobei die Temperatur- und Druckbedingungen der ersten Stufe ausgehend von Normaldruck und Raumtemperatur eingestellt werden. Besonders zweckmäßig hat es sich erwiesen, die Temperatur in zwei Stufen zu erhöhen und den Druck in zwei Stufen zu erniedrigen.

Die untere Grenze des einzustellenden Drucks hängt von den physikalischen Eigenschaften, wie Siedepunkte und Dampfdrücke, der Ausgangsverbindungen sowie der gebildeten Reaktionsprodukte ab und wird auch durch die Anlagenausstattung festgelegt. Ausgehend von Normaldruck kann innerhalb dieser Grenzwerte stufenweise mit von Stufe zu Stufe abnehmenden Drücken gearbeitet werden. Die obere Temperaturgrenze, üblicherweise 280°C, ist einzuhalten, um die Bildung von Zersetzungsprodukten, die u. a. farbschädigend wirken, zu vermeiden. Die untere Grenze der Temperaturstufen wird durch die Reaktionsgeschwindigkeit bestimmt, die noch ausreichend hoch sein muss, um die Veresterungsreaktion innerhalb einer vertretbaren Zeit abzuschließen. Innerhalb dieser Grenzwerte kann stufenweise mit von Stufe zu Stufe ansteigenden Temperaturen gearbeitet werden.

Die Veresterung kann mit stöchiometrischen Mengen Polyol und aliphatischer Monocarbonsäure vorgenommen werden. Vorzugsweise lässt man das Polyol jedoch mit überschüssiger Monocarbonsäure reagieren, die im Allgemeinen einen geringeren Siedepunkt aufweist als das eingesetzte Polyol und die bei der nachfolgenden Aufarbeitung des Rohesters auf einfache Weise destillativ abgetrennt werden kann. Die aliphatische Monocarbonsäure wird in einem 10 bis 50%-igen molaren, vorzugsweise in einem 20 bis 40%-igen molaren Überschuss je Mol zu veresternder Hydroxylgruppe des Polyols eingesetzt.

Das gebildete Reaktionswasser wird im Laufe der Veresterungsreaktion zusammen mit der überschüssigen Monocarbonsäure aus dem Reaktionsgefäß abdestilliert und in einen nachgeschalteten Phasentrenner geleitet, in dem sich Monocarbonsäure und Wasser je nach ihren Löslichkeitseigenschaften auftrennen. Zwischen Reaktionsgefäß und Phasentrenner kann ebenfalls eine Fraktionskolonne mit 1 bis 25, vorzugsweise 2 bis 10 und insbesondere 3 bis 6 theoretischen Böden installiert werden, in der die wasserangereicherte Fraktion über den Kolonnenkopf in den Phasentrenner geleitet wird und die monocarbonsäureangereicherte Fraktion über den Kolonnenboden in das Reaktionsgefäß zurückfließt.

Gegebenenfalls bildet die eingesetzte Monocarbonsäure mit Wasser unter den Reaktionsbedingungen auch ein Azeotrop und vermag als Schleppmittel das Reaktionswasser zu entfernen. Aus dem Wasseranfall kann der Reaktionsverlauf verfolgt werden. Das abgeschiedene Wasser wird aus dem Prozess entfernt, während die Monocarbonsäure aus dem Phasentrenner wieder in das Reaktionsgefäß zurückfließt. Die Zugabe eines weiteren organischen Lösungsmittels, wie Hexan, 1-Hexen, Cyclohexan, Toluol, Xylol oder Xylolisomerengemische, das die Aufgabe des Azeotropbildners übernimmt, ist nicht ausgeschlossen, jedoch auf wenige Ausnahmefälle beschränkt. Der Azeotropbildner kann bereits zu Beginn der Veresterungsreaktion oder nach dem Erreichen höherer Temperaturen zugesetzt werden. Wenn die theoretisch zu erwartende Wassermenge angefallen ist oder die Hydroxylzahl, beispielsweise bestimmt nach DIN 53240, unter einen festgelegten Wert gefallen ist, beendet man die Reaktion und beginnt mit der Aufarbeitung des Reaktionsansatzes.

Die Reaktion von Polyolen und aliphatischen Monocarbonsäuren kann ohne Einsatz eines Katalysators durchgeführt werden. Diese Variante der Umsetzung hat den Vorteil, dass man vermeidet, dem Reaktionsgemisch Fremdstoffe zuzuführen, die zu einer unerwünschten Verunreinigung des Polyolesters führen kann. Allerdings muss man dann im Allgemeinen höhere Reaktionstemperaturen einhalten, weil nur so gewährleistet ist, dass die Umsetzung mit ausreichender, d.h. wirtschaftlich vertretbarer Geschwindigkeit abläuft. Häufig kann die vorteilhafterweise im Überschuss eingesetzte aliphatischen Monocarbonsäure, die gleichzeitig Reaktionskomponente des Polyols ist, katalytisch wirksam sein, so dass die Veresterungsreaktion autokatalytisch abläuft.

Die Verwendung eines Katalysators, der die Umsetzung erleichtert und die Reaktionsgeschwindigkeit erhöht, lässt sich jedoch nicht immer vermeiden. Neben den üblichen Veresterungskatalysatoren, wie Broenstedtsäuren beispielsweise Schwefelsäure, Methansulfonsäure, para-Toluolsulfonsäure, haben sich besonders Lewissäuren enthaltend mindestens ein Element der Gruppen 4 bis 14 des Periodensystems der Elemente bewährt, die in fester oder flüssiger Form eingesetzt werden können. Unter dem Begriff Lewissäure im Sinne der Erfindung wird die allgemein übliche Definition für solche Elemente oder Verbindungen verstanden, die eine Elektronenlücke aufweisen, wie beispielsweise in Römpp's Chemie-Lexikon, 8. Auflage, Franck'sche Verlagshandlung 1983, Band 3, H-L ausgeführt. Zu den besonders geeigneten Lewissäuren, die als Katalysatoren in der Veresterungsreaktion eingesetzt werden können, zählen Titan, Zirkonium, Hafnium, Eisen, Zink, Bor, Aluminium oder Zinn, die als Element in fein verteilter Form oder bevorzugt in Form von Verbindungen verwendet werden. Geeignete Verbindungen sind beispielsweise Zinn(II)-oxid, Zinn(IV)oxid, Zinn-Carboxylate, wie Zinn(II)-2-ethylhexanoat, Zinn(II)-oxalat, Zinn(II)-acetat oder Zinn(IV)-acetat, Zinn(IV)-alkoholate, wie Tetra(methyl)stannat, Tetra(ethyl)stannat, Tetra(propyl)stannat, Tetra(iso-propyl)stannat oder Tetra(isobutyl)stannat, oder Organozinnverbindungen, wie Butylzinnmaleat oder Dibutylzinndilaurat. Zu den geeigneten Titanverbindungen zählen Alkoholate, wie Tetra(methyl)orthotitanat, Tetra(ethyl)orthotitanat, Tetra(propyl)orthotitanat, Tetra(iso-propyl)orthotitanat, Tetra(butyl)orthotitanat, Tetra(iso-butyl)ortho-titanat, Tetra(pentyl)orthotitanat oder Tetra(2-ethylhexyl)orthotitanat; Acylate, wie Hydroxytitanacetat, Hydroxytitanbutyrat oder Hydroxytitanpentanoat; Carboxylate wie Titan(IV)-acetat, Titan(IV)-propionat, Titan(IV)-butyrat, Titan(IV)-pentanoat oder Titan(IV)-2-ethylhexanoat; oder Chelate, wie Tetraethylenglykoltitanat oder Tetrapropylenglykoltitanat. Auch die ent-sprechenden Zirkonium- oder Hafniumverbindungen können mit Erfolg eingesetzt werden, wie Tetra(methyl)orthozirkonat, Tetra(ethyl)orthozirkonat, Tetra(propyl)orthozirkonat, Tetra(iso-propyl)orthozirkonat, Tetra(butyl)ortho-zirkonat, Tetra(iso-butyl)orthozirkonat, Tetra(pentyl)orthozirkonat oder Tetra(2-ethylhexyl)orthozirkonat.

Geeignet sind ebenfalls Borsäure sowie Borsäureester, wie Borsäuretrimethylester, Borsäuretriethylester, Borsäuretripropylester, Borsäuretriisopropylester, Borsäuretributylester oder Borsäuretriisobutylester.

Ebenfalls geeignet sind Aluminiumoxid, Aluminiumhydroxid, Aluminiumcarboxylate, wie Aluminiumacetat oder Aluminiumstearat, oder Aluminiumalkoholate wie Aluminiumtributylat, Aluminium-tri-sec.-butylat, Aluminium-tri-tert.-butylat oder Aluminium-tri-isopropylat.

Auch Zinkoxid, Zinksulfat und Zinkcarboxylate wie Zinkacetat Dihydrat oder Zinkstearat, und Eisen(II)acetat oder Eisen(III)hydroxid-oxid können als Katalysatoren eingesetzt werden.

Der Katalysator kann dem Reaktionsgemisch bereits zu Beginn zugesetzt werden oder erst nachträglich unter Beachtung von Sicherheitsmaßnahmen bei erhöhter Temperatur, wenn beispielsweise die Abtrennung des Reaktionswassers eingesetzt hat. Der Katalysator kann dabei in einer Portion oder portionsweise zugesetzt werden. Besonders empfehlenswert ist, gegen Ende der Veresterungsreaktion noch eine Restmenge an Katalysator zuzusetzen.

Die Menge des zugesetzten Veresterungskatalysators beträgt 1X10⁻⁵ bis 20 mol%, vorzugsweise 0,01 bis 5 mol-%, insbesondere 0,01 bis 2 mol%, bezogen auf die im Unterschuss zugesetzte Ausgangsverbindung, zweckmäßigerweise bezogen auf Polyol. Bei höheren Katalysatormengen ist mit Spaltungsreaktionen der Polyolester zu rechnen.

Besonders bei der Herstellung von Polyolestern auf Basis von Etherdiolen, wie zum Beispiel Triethylenglykol oder Tetraethylenglykol, ist bei dem Einsatz hoher Katalysatorkonzentrationen gegen Reaktionsende und in der Phase des Umsatzes letzter Reste an freien Hydroxylgruppen eine verstärkte Spaltung der Etherkette zu befürchten, so dass in diesem Falle die Reaktionstemperatur oder der anzulegende Druck anzupassen sind. Je höher die gewählte Katalysatorkonzentration ist, desto niedriger ist im Allgemeinen die Reaktionstemperatur oder der anzulegende Druck zu wählen und es ist nach einem optimierten Temperatur- und Druckprofil zu arbeiten.

In einer Ausgestaltung des erfindungsgemäßen Verfahrens kann die Veresterung in Gegenwart eines Adsorbens durchgeführt werden. Man verwendet dabei poröse, großflächige feste Materialien, die üblicherweise in der chemischen Praxis sowohl im Labor als auch in technischen Anlagen eingesetzt werden. Beispiele für solche Materialien sind oberflächenreiche Polykieselsäuren wie Silicagele (Kiesel-Xerogele), Kieselgel, Kieselguhr, oberflächenreiche Aluminiumoxide und Aluminiumoxidhydrate, mineralische Materialien, wie Tone oder Carbonate, oder Aktivkohle. Besonders bewährt hat sich Aktivkohle. Im Allgemeinen ist das Adsorbens feinteilig in der Reaktionslösung suspendiert, die durch intensives Rühren oder durch Einleiten eines Inertgases bewegt wird. Dadurch wird ein inniger Kontakt zwischen der flüssigen Phase und dem Adsorbens erreicht. Die Menge des Adsorbens kann weitgehend frei und somit den individuellen Erfordernissen entsprechend eingestellt werden. Bezogen auf 100 Gewichtsteile des flüssigen Reaktionsansatzes bewährt es sich 0,1 bis 5, vorzugsweise 0,5 bis 1,5 Gewichtsteile des Adsorbens einzusetzen.

Das nach Beendigung der Umsetzung anfallende Reaktionsgemisch enthält neben dem Polyolester als erwünschtem Reaktionsprodukt gegebenenfalls nicht umgesetzte Ausgangsstoffe, insbesondere noch überschüssige aliphatische Monocarbonsäure, sofern nach der bevorzugten Ausgestaltung des erfindungsgemäßen Verfahrens mit einem Monocarbonsäureüberschuss gearbeitet wird. Üblicherweise destilliert man zunächst unumgesetzte und im Überschuss vorliegende Ausgangsverbindungen ab, zweckmäßigerweise unter Anlegen eines verminderten Drucks. Falls unter Zugabe von festen Veresterungskatalysatoren gearbeitet wird, beispielsweise mit Zinn(II)oxid, Zinkoxid oder Eisen(III)hydroxidoxid, werden nach beendeter Veresterungsreaktion im Zuge der weiteren Aufarbeitung die Feststoffe abgetrennt. Werden die Veresterungskatalysatoren als flüssige Verbindungen zugesetzt, beispielsweise Tetra(iso-propyl)orthotitanat oder Tetra(butyl)orthotitanat, die nach beendeter Veresterungsreaktion im Reaktionsgemisch noch gelöst vorliegen, so werden diese Verbindungen im Zuge der weiteren Aufarbeitung in schwer lösliche Umwandlungsprodukte überführt, beispielsweise durch die Behandlung mit Wasser oder Wasserdampf, die dann abfiltriert werden können.

Gegebenenfalls wird der Rohester von Feststoffen und gegebenenfalls vom Adsorbens, falls die Veresterung in Gegenwart eines Adsorbens durchgeführt wurde, filtriert. Die Filtration erfolgt in konventionellen Filtrierapparaturen bei normaler Temperatur oder bei Temperaturen bis 120°C, gegebenenfalls in Gegenwart gängiger Filtrierhilfsmittel wie Cellulose, Kieselgel, Kieselgur oder Holzmehl.

Es folgt eine Behandlung mit Wasserdampf, die beispielsweise in einfacher Form durch Einleiten von Wasserdampf in das Rohprodukt erfolgen kann. Die Wasserdampfbehandlung kann in Gegenwart oder in Abwesenheit von Feststoffen erfolgen, je nachdem, ob vor der Wasserdampfbehandlung ein Filtrationsschritt vorgenommen wird. Durch die Wasserdampfbehandlung können ebenfalls die Farbzahl und die Farbstabilität des Rohesters verbessert werden.

Die Wasserdampfbehandlung wird im Allgemeinen bei Normaldruck durchgeführt, obwohl die Anwendung eines leichten Unterdrucks zweckmäßigerweise bis 400 hPa nicht ausgeschlossen ist. Die Wasserdampfbehandlung wird im Allgemeinen bei Temperaturen von 120 bis 260°C, vorzugsweise von 150 bis 220°C und insbesondere von 170 bis 200°C, durchgeführt und richtet sich auch nach den physikalischen Eigenschaften der jeweils herzustellenden Polyolester.

Bei dem Prozessschritt der Wasserdampfbehandlung erweist es sich als zweckmäßig, während der Aufheizperiode bis zum Erreichen der Arbeitstemperatur möglichst schonend vorzugehen, um den Rohester auf die erforderliche Temperatur für die Wasserdampfbehandlung zu erhitzen.

Die Dauer der Wasserdampfbehandlung lässt sich durch Routineversuche ermitteln und sie wird im Allgemeinen über einen Zeitraum von 0,5 bis 10 Stunden, vorzugsweise 1 bis 5 Stunden durchgeführt. Eine zu lange Wasserdampfbehandlung führt zu einer ungewünschten Erhöhung der Farbzahl des Polyolesters und ist daher zu vermeiden. Auch beobachtet man eine verstärkte Abbaureaktion des Polyolesters zu sauer reagierenden Verbindungen, deren Gehalt sich in einem Anstieg der Neutralisationszahl oder Säurezahl beispielsweise bestimmt nach DIN EN ISO 3682/ASTM D 1613 zeigt.

Im Anschluss an die gegebenenfalls durchgeführte Filtration kann optional unmittelbar vor der Wasserdampfbehandlung der Rohester zur Farbaufhellung mit einer oxidierenden Verbindung behandelt werden, sofern die Farbzahl des Rohesters dies erfordert. Als oxidierende Verbindungen eignen sich peroxidische Verbindungen oder Ozon sowie ozonhaltige Gase. Insbesondere geeignet ist eine wässrige Lösung von Wasserstoffperoxid mit einem Wasserstoffperoxidgehalt von mehr als 10 Gew.-%, vorzugsweise 30 bis 50 Gew.-%. Üblicherweise wird die peroxidische Verbindung mit einem Wirkgehalt von 0,05 bis 1,0 Gew.-%, vorzugsweise von 0,08 bis 0,3 Gew.-%, bezogen auf die Gesamtmenge des zu behandelnden Rohesters, appliziert. Bei zu hohen Wirkkonzentrationen ist mit unkontrollierten Abbaureaktionen der Polyolester zu rechnen.

Die Behandlung mit peroxidischen Verbindungen erfolgt im Allgemeinen bei Temperaturen von 70 bis 160°C, vorzugsweise 100 bis 120°C über einen Behandlungszeitraum von 0,5 bis 3 Stunden. Bei zu langen Behandlungszeiten kann es auf Grund des anwesenden Wassers und des Oxidationsmittels zu einer verstärkten Esterspaltung und zu einem unkontrollierten Abbau des Polyolestergerüstes kommen.

Falls Ozon oder ozonhaltige Gase zur Farbaufhellung benutzt werden, wird Ozon in einer Menge von 0,01 bis 5 Gramm, vorzugsweise von 0,2 bis 0,8 Gramm, pro Liter Polyolester eingesetzt. Höhere Ozonmengen sind aufgrund verstärkt einsetzender Abbaureaktionen des Polyolestergerüstes nicht zu empfehlen. Wird Ozon im Gemisch mit anderen Gasen, vorzugsweise im Gemisch mit Sauerstoff, eingesetzt, beträgt die Ozonkonzentration zweckmäßigerweise 2 bis 200, vorzugsweise 10 bis 100 Gramm Ozon pro m³ Gasgemisch. Die Behandlung mit Ozon erfolgt im Allgemeinen bei Temperaturen von 20 bis 100°C, vorzugsweise 30 bis 80°C und über einen Zeitraum von 20 bis 90 Minuten.

Die jeweiligen Bedingungen für die Behandlung mit der oxidierenden Verbindung sind auf den jeweiligen Polyolester zuzuschneiden, um eine optimale Entfärbung auf der einen Seite zu erzielen aber auf der anderen Seite Abbaureaktionen des Polyolesters möglichst zu vermeiden. Insbesondere bei Polyolestern auf Basis von Etherdiolen, wie zum Beispiel Triethylenglykol oder Tetraethylenglykol, kann ein verstärkter Abbau des Ethergerüstes einsetzen, wenn die Bedingungen bei der Behandlung mit der oxidierenden Verbindung wie Temperatur, Einwirkdauer und Wirkkonzentration nicht gezielt auf den jeweiligen Polyolester eingestellt werden.

Nach der Behandlung mit den oxidierenden Reagentien wird der Rohester unmittelbar anschließend ohne weitere Zwischenschritte der Behandlung mit Wasserdampf unterzogen, die beispielsweise in einfacher Form durch Einleiten von Wasserdampf in das Rohprodukt erfolgen kann. Ein Vorteil der Wasserdampfbehandlung ist, dass in ihrem Verlauf überschüssige oxidierende Verbindungen zerstört werden können und Reste der Ausgangsverbindungen mit dem Wasserdampf entfernt werden können. Auch größere Mengen noch vorhandenen Wassers werden durch die Wasserdampfbehandlung ausgetrieben. Gleichzeitig können durch diese Maßnahme die Farbzahl und die Farbstabilität des Rohesters verbessert werden.

Die Bedingungen der Wasserdampfbehandlung, die sich unmittelbar an die Behandlung mit den oxidierenden Reagentien anschließt, entsprechen den zuvor genannten Bedingungen für die Wasserdampfbehandlung des Rohesters nach der gegebenenfalls durchgeführten Filtration. Dabei ist zu beachten, dass bei einer zu kurzen Behandlungsdauer die Zerstörung der überschüssigen oxidierenden Verbindungen und gebildeter Spuren von organischen Peroxiden nicht vollständig ist und der gewünschte Polyolester noch eine zu hohe unerwünschte Peroxidzahl, ausgedrückt in Milliäquivalent Sauerstoff pro Kilogramm Produkt und bestimmt nach ASTM E 298, aufweist. Auch wird bei zu kurzer Behandlungsdauer nur ein geringer vorteilhafter Effekt auf die Farbzahl des Polyolesters beobachtet.

Wie bei der Behandlung mit den oxidierenden Verbindungen sind auch bei der sich unmittelbar anschließenden Wasserdampfbehandlung die Bedingungen, wie Temperatur, Druck und Dauer gezielt auf den jeweiligen Polyolester einzustellen, um ein optimales Ergebnis in Bezug auf die Farbzahl des Polyolesters zu erzielen und um Restgehalte an Ausgangsverbindungen, Wasser sowie an Peroxidspuren möglichst zu minimieren und gleichzeitig Abbaureaktionen zu unterdrücken. Insbesondere bei Polyolestern auf Basis von Etherdiolen, wie zum Beispiel Triethylenglykol oder Tetraethylenglykol, sind die Bedingungen bei der Wasserdampfbehandlung auf den jeweiligen Polyolester genau zuzuschneiden, um den unerwünschten Abbau der Etherkette zu unterbinden.

Es fällt als Rückstand ein aufgehellter Polyolester an, der anschließend getrocknet wird, beispielsweise in dem man ein inertes Gas durch das Produkt bei erhöhter Temperatur leitet. Auch Wasserdampf kann zusätzlich eingeleitet werden, um den Trocknungsvorgang zu unterstützen. Es kann auch bei erhöhter Temperatur gleichzeitig ein Unterdruck angelegt und gegebenenfalls ein inertes Gas durch das Produkt geleitet werden. Auch ohne Einwirken eines inerten Gases kann nur bei erhöhter Temperatur oder nur bei geringerem Druck gearbeitet werden. Die jeweiligen Trocknungsbedingungen, wie Temperatur, Druck und Dauer lassen sich durch einfache Vorversuche ermitteln. Im Allgemeinen arbeitet man bei Temperaturen im Bereich von 80 bis 250°C, vorzugsweise 100 bis 180°C und bei Drücken von 0,2 bis 500 hPa, vorzugsweise 1 bis 200 hPa und insbesondere 1 bis 20 hPa. Durch die Trocknung, gegebenenfalls zusammen mit dem Einleiten von Wasserdampf, werden Reste an Ausgangsverbindungen, beispielsweise Monocarbonsäure, und Wasser entfernt. Der gereinigte Polyolester verbleibt während der Trocknung als Rückstand im Reaktionsgefäß. Es werden hellfarbige Polyolester erhalten, die auch den übrigen Spezifikationswerten wie Wassergehalt, Restsäuregehalt, Restgehalt an Monoester sowie an Katalysatorbestandteilen, falls die Veresterung unter Katalyse erfolgte, genügen.

Der flüchtige Nebenstrom aus der Wasserdampfbehandlung wird abgeführt und kondensiert. Das Kondensat trennt sich in eine wässrige und in eine organische Phase. Anschließend wird die organische Phase, die einen Polyolestergehalt im Allgemeinen von 40 bis 80 Gew.-% aufweist, zur Aufkonzentrierung einer weiteren Wasserdampfbehandlung, wie zuvor beschrieben, unterzogen. Es wird ein aufkonzentrierter Rückstand mit einem Polyolestergehalt im Allgemeinen von mehr als 80 Gew.-%, vorzugsweise mehr als 90 Gew.-%, jeweils bezogen auf den flüssigen Rückstand, erhalten, der in den Produktionsprozess zur Polyolesterherstellung zurückgeführt wird. Durch die aus dem flüchtigen Nebenstrom zusätzlich zurückgewonnene Polyolestermenge können ohne zusätzlichen Investitionsaufwand die Kapazität der Produktionsanlage und die Rohstoffeffizienz erhöht werden.

Als Polyole geeignet sind Verbindungen der allgemeinen Formel (II)

H-(-O-[-CR¹R²-]ₘ-)ₒ-OH (II)

in der R¹ und R² unabhängig voneinander Wasserstoff, einen Alkylrest mit 1 bis 5 Kohlenstoffatomen, vorzugsweise Methyl, Ethyl oder Propyl, oder einen Hydroxyalkylrest mit 1 bis 5 Kohlenstoffatomen, vorzugsweise der Hydroxymethylrest, m eine ganze Zahl von 1 bis 10, vorzugsweise 1 bis 8 und insbesondere 1, 2, 3 oder 4, o eine ganze Zahl von 2 bis 15, vorzugsweise 2 bis 8 und insbesondere 2, 3, 4 oder 5 bedeuten.

Als Polyole, die nach dem erfindungsgemäßen Verfahren zu hellfarbigen Polyolestern umgesetzt werden können, eignen sich beispielsweise Di-Trimethylolpropan oder Di-Pentaerythrit.

Als weitere Polyole kommen die Oligomeren von Ethylenglykol und 1,2-Propylenglykol, insbesondere die Etherdiole Di-, Tri- und Tetraethylenglykol oder Dipropylenglykol, Tripropylenglykol oder Tetrapropylenglykol in Betracht. Ethylen- und Propylenglykole sind industriell produzierte Chemikalien. Basissubstanz zu ihrer Herstellung ist Ethylenoxid und Propylenoxid, aus dem man 1,2-Ethylenglykol und 1,2-Propylenglykol durch Erhitzenmit Wasser unter Druck gewinnt. Diethylenglykol erhält man durch Ethoxylierung aus Ethylenglykol. Triethylenglykol fällt, wie Tetraethylenglykol, als Nebenprodukt bei der Hydrolyse von Ethylenoxid zur Herstellung von Ethylenglykol an. Beide Verbindungen können auch durch Umsetzung von Ethylenglykol mit Ethylenoxid synthetisiert werden. Dipropylenglykol, Tripropylenglykol, Tetrapropylenglykol und höhere Propoxylierungsprodukte sind aus der mehrfachen Addition von Propylenoxid an 1,2-Propylenglykol zugänglich.

Zur Gewinnung von hellfarbigen Polyolestern nach dem erfindungsgemäßen Prozess setzt man lineare oder verzweigte, aliphatische Monocarbonsäuren mit 3 bis 20 Kohlenstoffatomen im Molekül ein, ausgewählt aus der Gruppe von Propionsäure, n-Buttersäure, Isobuttersäure, n-Pentansäure, 2-Methylbuttersäure, 3-Methylbuttersäure, 2-Methylpentansäure, n-Hexansäure, 2-Ethylbuttersäure, n-Heptansäure, 2-Methylhexansäure, Cyclohexancarbonsäure, 2-Ethylhexansäure, n-Nonansäure, 2-Methyloctansäure, Isononansäure, 3,5,5-Trimethylhexansäure, 2-Propylheptansäure, 2-Methylundecansäure, Isoundecancarbonsäure, Tricyclodecancarbonsäure und Isotridecancarbonsäure. Besonders bewährt hat sich das neue Verfahren für die Herstellung von Polyolestern der oligomeren Ethylenglykole sowie der oligomeren Propylenglykole mit C₄- bis C₁₃- bzw. C₅- bis C₁₀-Monocarbonsäuren sowie zur Herstellung von Polyolestern auf Basis von Di-Trimethylolpropan.

Die Polyolester des Ethylenglykols sowie seiner Oligomeren eignen sich hervorragend als Weichmacher für alle gängigen hochpolymeren thermoplastischen Stoffe. Besonders bewährt haben sie sich als Zusatz zu Polyvinylbutyral, das mit Glykolestern versetzt als Zwischenschicht zur Herstellung von Mehrschichten- oder Verbundgläsern verwendet wird. Sie können ebenfalls als Koaleszenzmittel oder Filmbildehilfsmittel in wässrigen Dispersionen von Kunststoffen, die als Beschichtungsmittel vielfältige Anwendung finden, eingesetzt werden. Nach dem erfindungsgemäßen Herstellungsverfahren lässt sich auf einfache Weise die Ausbeute an Polyolestern steigern, die ebenfalls den erforderlichen Qualitätsansprüchen, wie geringem Geruch, einer geringen Farbzahl, einer geringen Säurezahl und geringen Katalysatorverunreinigungen genügen. Besonders eignet sich das erfindungsgemäße Verfahren zur Herstellung von Triethylenglykol-di-2-ethylhexanoat (3G8 Ester), Tetraethylenglykol-di-n-heptanoat (4G7 Ester), Triethylenglykol-di-2-ethylbutyrat (3G6 Ester), Triethylenglykol-di-n-heptanoat (3G7 Ester) oder Tetraethylenglykol-di-2-ethylhexanoat (4G8 Ester).

Das erfindungsgemäße Verfahren kann kontinuierlich oder absatzweise in den für die chemische Technik typischen Reaktionsapparaten durchgeführt werden. Bewährt haben sich Rührkessel, auch als Rührkesselkaskade, oder Reaktionsrohre, wobei die absatzweise Reaktionsführung die bevorzugte ist.

In den folgenden Beispielen wird das erfindungsgemäße Verfahren näher erläutert.

### Ausführungsbeispiele:

Für die Versuche zur Gewinnung von Triethylenglykol-di-2-ethylhexanoat aus den Nebenströmen kam rohes Triethylenglykol-di-2-ethyl-hexanoat zum Einsatz, das durch Veresterung von Triethylenglykol mit einer 2,4 molaren Menge an 2-Ethylhexansäure unter Zusatz von 0,025 mol-% Tetra(isopropyl)orthotitanat als Katalysator, bezogen auf Triethylenglykoleinsatz, und 0,4 Gew.-% Aktivkohle, bezogen auf den Reaktionsansatz, erhalten wurde.

Zur Aufarbeitung des rohen Triethylenglykol-di-2-ethylhexanoats wurde die überschüssige 2-Ethylhexansäure abdestilliert, der Titankatalysator in schwerlösliche Folgeprodukte mittels Wasserdampfdestillation überführt und der Rohester von Feststoffen abfiltiert. Nach Zugabe einer 30%-igen wässrigen Wasserstoffperoxidlösung in einer Menge von 0,1 Gew.-% Wasserstoffperoxid, absolut, wurde der Ansatz über eine Stunde bei 120°C gerührt. Die sich anschließende Wasserdampfdestillation erfolgte über einen Zeitraum von einer Stunde bei einer Temperatur von 200°C unter Normaldruck.

### Beispiel 1:

Das nach der Wasserstoffperoxidbehandlung erhaltene, flüchtige Wasserdampfdestillat wurde kondensiert und die sich abscheidende organische Phase von der wässrigen Phase getrennt. Die abgetrennte organische Phase oder der Nebenstrom wurde gemäß den Beispielen einer weiteren Wasserdampfbehandlung unterzogen. Die gaschromatographisch ermittelten Gehalte an Triethylenglykol-di-2-ethylhexanoat (in Gew.-%) sowie die Bedingungen der weiteren Wasserdampfbehandlung sind in der nachfolgenden Tabelle 1 zusammengestellt.

**Tabelle 1: Wasserdampfbehandlung des bei der Herstellung von Triethylenglykol-di-2-ethylhexanoat (3G8 Ester) erhaltenen wässrigen, flüchtigen Wasserdampfdestillats (Nebenstrom)**

| | 3G8 Ester Gehalt (Gew.-%) | Farbzahl (nach Hazen DIN ISO 6271) |
|---|---|---|
| Einsatz | 77,5 | 14 |
| 1 h 150°C | 82,7 | 20 |
| 4 h 150°C | 86,8 | 27 |
| 1 h 190°C | 85,4 | 28 |
| 2 h 190°C | 88,8 | 49 |
| 3 h 190°C | 91,0 | 87 |
| 4 h 190°C | 92,4 | 101 |
| 1 h 150 °C, 1 h 180°C, 2 h 200°C | 92,4 | 69 |

Aus den Angaben der Tabelle 1 zeigt sich eine starke Temperaturabhängigkeit des Triethylenglykol-di-2-ethylhexanoatgehaltes. Um die leichtsiedenden Komponenten möglichst rasch zu entfernen, sind höhere Temperaturen effektiver. Allerdings steigt die Farbzahl bei höheren Temperaturen ebenfalls stark an. Wird ein angepasstes Temperaturprofil gefahren, so kann ein hoher Gehalt an Triethylenglykol-di-2-ethylhexanoat sowie auch eine niedrigere Farbzahl erreicht werden.

### Beispiel 2:

Der erzielbare Triethylenglykol-di-2-ethylhexanoat-Gehalt bzw. die dafür notwendige Zeitdauer hängen stark von der Zusammensetzung des Nebenstromes ab. Wird im Vergleich zu Beispiel 1 von einem geringeren Triethylenglykol-di-2-ethylhexanoat-Gehalt im Nebenstrom ausgegangen, so ist der bei gleicher Temperatur und Zeitdauer erzielbare Polyolester-Gehalt ebenfalls geringer. Um einen annehmbaren Polyolester-Gehalt sowie eine annehmbare Farbzahl zu erzielen, muss die Behandlung mit Wasserdampf hinsichtlich Temperatur und Zeitdauer angepasst werden. Durch eine nachträgliche Behandlung mit einer 30 Gew.-% Wasserstoffperoxidlösung in einer Menge von 0,1 Gew.-% absolut, bezogen auf die Polyolestermenge, über einen Zeitraum von einer Stunde und bei einer Temperatur von 120°C, entsprechend dem aus DE 10 2009 048773 A1 bekannten Verfahren, kann die Farbzahl deutlich reduziert werden.

**Tabelle 2: Abhängigkeit des erzielbaren Triethylenglykol-di-2-ethylhexanoat-Gehalts (3G8 Ester-Gehalts, gaschromatographisch ermittelt) von der Zusammensetzung des Nebenstromes und Nachbehandlung mit Wasserstoffperoxid (30%-ige wässrige Lösung, 1 Gew.-% absolut bezogen auf Polyolester, eine Stunde; 120°C**

| | 3G8 Ester Gehalt (Gew.-%) | Farbzahl* | Farbzahl* nach H₂O₂-Behandlung |
|---|---|---|---|
| Einsatz | 62,1 | 14 | |
| 4 h 190°C | 83,0 | 136 | 54 |
| 2 h 150°C, 2 h 190°C | 80,9 | 86 | 19 |
| 1 h 150°C, 1 h 180°C, 2 h 210°C | 86,7 | 175 | 55 |
| 1 h 150°C, 1 h 170°C, 1 h 190°C, 1 h 210°C | 84,8 | 228 | 50 |

| | | | |
|---|---|---|---|
| *nach Hazen DIN ISO 6271 | | | |

## Patentansprüche

1. Verfahren zur Gewinnung von mit Polyolestern angereicherten Produktströmen aus den Nebenströmen der Polyolesterherstellung, umfassend
a) die Umsetzung von Polyolen der allgemeinen Formel (II)
H-(-O-[-CR¹R²-]ₘ-)ₒ-OH (II)
in der R¹ und R² unabhängig voneinander Wasserstoff, einen Alkylrest mit 1 bis 5 Kohlenstoffatomen, vorzugsweise Methyl, Ethyl oder Propyl, oder einen Hydroxyalkylrest mit 1 bis 5 Kohlenstoffatomen, vorzugsweise der Hydroxymethylrest, m eine ganze Zahl von 1 bis 10, vorzugsweise 1 bis 8 und insbesondere 1, 2, 3 oder 4, o eine ganze Zahl von 2 bis 15, vorzugsweise 2 bis 8 und insbesondere 2, 3, 4 oder 5 bedeuten, mit linearen oder verzweigten aliphatischen Monocarbonsäuren mit 3 bis 20 Kohlenstoffatomen ausgewählt aus der Gruppe von Propionsäure, n-Buttersäure, Isobuttersäure, n-Pentansäure, 2-Methylbuttersäure, 3-Methylbuttersäure, 2-Methylpentansäure, n-Hexansäure, 2-Ethylbuttersäure, n-Heptansäure, 2-Methylhexansäure, 2-Ethylhexansäure, n-Nonansäure, 2-Methyloctansäure, Isononansäure, 3,5,5-Trimethylhexansäure oder 2-Propylheptansäure unter Entfernung des gebildeten Wassers;
b) die Abtrennung unumgesetzter Ausgangsverbindungen von dem gebildeten Rohester;
c) die Behandlung des gemäß Schritt b) erhaltenen Rohesters mit Wasserdampf unter Bildung eines flüchtigen Nebenstromes; und
d) die Abtrennung des in Schritt c) erhaltenen flüchtigen Nebenstromes,
**dadurch gekennzeichnet, dass** der in Schritt d) erhaltene flüchtige Nebenstrom in eine wässrige Phase und in eine organische Phase getrennt wird und die abgetrennte organische Phase einer weiteren Behandlung mit Wasserdampf unterzogen wird und ein mit Polyolestern angereicherter Produktstrom als Rückstand gewonnen wird.

2. Verfahren gemäß Anspruch 1, **dadurch gekennzeichnet, dass** man die weitere Behandlung mit Wasserdampf bei einer Temperatur von 120 bis 260°C, vorzugsweise von 150 bis 220°C, über einen Zeitraum von 1 bis 10 Stunden, vorzugsweise 1 bis 5 Stunden, durchführt.

3. Verfahren gemäß Anspruch 1 oder 2, **dadurch gekennzeichnet, dass** man die weitere Wasserdampfbehandlung stufenweise mit von Stufe zu Stufe ansteigenden Temperaturen durchführt.

4. Verfahren gemäß einem oder mehreren Ansprüchen 1 bis 3, **dadurch gekennzeichnet, dass** die weitere Wasserdampfbehandlung in Gegenwart eines Adsorbens durchgeführt wird.

5. Verfahren gemäß einem oder mehreren der Ansprüche 1 bis 4, **dadurch gekennzeichnet, dass** sich an die weitere Wasserdampfbehandlung eine Behandlung mit Wasserstoffperoxid und unmittelbar folgend eine erneute Wasserdampfbehandlung anschließt.

6. Verfahren gemäß einem oder mehreren der Ansprüche 1 bis 5, **dadurch gekennzeichnet, dass** man unmittelbar vor Schritt c) den Rohester mit oxidierenden Verbindungen behandelt.

7. Verfahren gemäß Anspruch 6, **dadurch gekennzeichnet, dass** man als oxidierende Verbindungen peroxidische Verbindung, Ozon oder ozonhaltige Gase verwendet.

8. Verfahren gemäß Anspruch 7, **dadurch gekennzeichnet, dass** man als peroxidische Verbindung Wasserstoffperoxid verwendet.

9. Verfahren gemäß einem oder mehreren der Ansprüche 1 bis 8, **dadurch gekennzeichnet, dass** man in Schritt a) die Polyole mit den Monocarbonsäuren in Gegenwart eines Katalysators umsetzt.

10. Verfahren gemäß Anspruch 9, **dadurch gekennzeichnet, dass** man als Katalysator Titan, Zirkonium, Hafnium, Eisen, Zink, Bor, Aluminium oder Zinn als Elemente oder in Form ihrer Verbindungen verwendet.

11. Verfahren gemäß einem oder mehreren der Ansprüche 1 bis 10, **dadurch gekennzeichnet, dass** man in Schritt a) die Polyole mit den Monocarbonsäuren in Gegenwart eines Adsorbens umsetzt.

12. Verfahren gemäß Anspruch 11, **dadurch gekennzeichnet, dass** man als Adsorbens Silicagel, Kieselgel, Kieselguhr, Aluminiumoxid, Aluminiumoxidhydrate, Tone, Carbonate oder Aktivkohle verwendet.

13. Verfahren gemäß einem oder mehreren der Ansprüche 1 bis 12, **dadurch gekennzeichnet, dass** man als Polyole Di-Trimethylolpropan, Di-Pentaerythrit, Diethylenglykol, Triethylenglykol, Tetraethylenglykol, Dipropylenglykol, Tripropylenglykol oder Tetrapropylenglykol verwendet.

14. Verfahren gemäß einem oder mehreren der Ansprüche 1 bis 13 zur Herstellung von Triethylenglykol-di-2-ethylhexanoat, Tetraethylenglykol-di-n-heptanoat, Triethylenglykol-di-2-ethylbutyrat, Triethylenglykol-di-n-heptanoat oder Tetraethylenglykol-di-2-ethylhexanoat.

## Claims

1. Process for obtaining product streams enriched with polyol esters from the secondary streams from polyol ester preparation, comprising
a) the reaction of polyols of the general formula (II)
H-(-O-[-CR¹R²-]ₘ-)ₒ-OH (II)
in which R¹ and R² are each independently hydrogen, an alkyl radical having 1 to 5 carbon atoms, preferably methyl, ethyl or propyl, or a hydroxyalkyl radical having 1 to 5 carbon atoms, preferably the hydroxymethyl radical, m is an integer from 1 to 10, preferably 1 to 8 and especially 1, 2, 3 or 4, o is an integer from 2 to 15, preferably 2 to 8 and especially 2, 3, 4 or 5, with linear or branched aliphatic monocarboxylic acids having 3 to 20 carbon atoms, selected from the group of propionic acid, n-butyric acid, isobutyric acid, n-pentanoic acid, 2-methylbutyric acid, 3-methylbutyric acid, 2-methylpentanoic acid, n-hexanoic acid, 2-ethylbutyric acid, n-heptanoic acid, 2-methylhexanoic acid, 2-ethylhexanoic acid, n-nonanoic acid, 2-methyloctanoic acid, isononanoic acid, 3,5,5-trimethylhexanoic acid and 2-propylheptanoic acid, with removal of the water formed;
b) the removal of unconverted starting compounds from the crude ester formed;
c) the treatment of the crude ester obtained in step b) with steam to form a volatile secondary stream; and
d) the removal of the volatile secondary stream obtained in step c),
**characterized in that** the volatile secondary stream obtained in step d) is separated into an aqueous phase and an organic phase and the organic phase removed is subjected to a further treatment with steam and a product stream enriched with polyol esters is obtained as residue.

2. Process according to Claim 1, **characterized in that** the further treatment with steam is conducted at a temperature of 120 to 260°C, preferably of 150 to 220°C, over a period of 1 to 10 hours, preferably 1 to 5 hours.

3. Process according to Claim 1 or 2, **characterized in that** the further steam treatment is conducted in stages with temperatures rising from stage to stage.

4. Process according to one or more of Claims 1 to 3, **characterized in that** the further steam treatment is conducted in the presence of an adsorbent.

5. Process according to one or more of Claims 1 to 4, **characterized in that** the further steam treatment is followed by a treatment with hydrogen peroxide, immediately followed by another steam treatment.

6. Process according to one or more of Claims 1 to 5, **characterized in that** the crude ester is treated with oxidizing compounds immediately prior to step c) .

7. Process according to Claim 6, **characterized in that** the oxidizing compounds used are peroxidic compounds, ozone or ozone-containing gases.

8. Process according to Claim 7, **characterized in that** the peroxidic compound used is hydrogen peroxide.

9. Process according to one or more of Claims 1 to 8, **characterized in that** the polyols are reacted with the monocarboxylic acids in step a) in the presence of a catalyst.

10. Process according to Claim 9, **characterized in that** the catalyst used is titanium, zirconium, hafnium, iron, zinc, boron, aluminum or tin as elements or in the form of their compounds.

11. Process according to one or more of Claims 1 to 10, **characterized in that** the polyols are reacted with the monocarboxylic acids in step a) in the presence of an adsorbent.

12. Process according to Claim 11, **characterized in that** the adsorbent used is silica gel, kieselguhr, alumina, alumina hydrates, clays, carbonates or activated carbon.

13. Process according to one or more of Claims 1 to 12, **characterized in that** the polyols used are ditrimethylolpropane, dipentaerythritol, diethylene glycol, triethylene glycol, tetraethylene glycol, dipropylene glycol, tripropylene glycol or tetrapropylene glycol.

14. Process according to one or more of Claims 1 to 13 for preparing triethylene glycol di-2-ethylhexanoate, tetraethylene glycol di-n-heptanoate, triethylene glycol di-2-ethylbutyrate, triethylene glycol di-n-heptanoate or tetraethylene glycol di-2-ethylhexanoate.

## Revendications

1. Procédé pour l'obtention de flux de produits enrichis en polyolesters à partir des flux secondaires de la préparation de polyolesters, comprenant
a) la transformation de polyols de formule générale (II)
H-(-O-[-CR¹R²-]ₘ-)ₒ-OH (II)
dans laquelle R¹ et R² signifient, indépendamment l'un de l'autre, hydrogène, un radical alkyle comprenant 1 à 5 atomes de carbone, de préférence méthyle, éthyle ou propyle, ou un radical hydroxyalkyle comprenant 1 à 5 atomes de carbone, de préférence le radical hydroxyméthyle, m vaut un nombre entier de 1 à 10, de préférence de 1 à 8 et en particulier 1, 2, 3 ou 4, o vaut un nombre entier de 2 à 15, de préférence de 2 à 8 et en particulier 2, 3, 4 ou 5, avec des acides monocarboxyliques linéaires ou ramifiés, aliphatiques, comprenant 3 à 20 atomes de carbone, choisis dans le groupe formé par l'acide propionique, l'acide n-butyrique, l'acide isobutyrique, l'acide n-pentanoïque, l'acide 2-méthylbutyrique, l'acide 3-méthylbutyrique, l'acide 2-méthylpentanoïque, l'acide n-hexanoïque, l'acide 2-éthylbutyrique, l'acide n-heptanoïque, l'acide 2-méthylhexanoïque, l'acide 2-éthylhexanoïque, l'acide n-nonanoïque, l'acide 2-méthyloctanoïque, l'acide isononanoïque, l'acide 3,5,5-triméthylhexanoïque ou l'acide 2-propylheptanoïque avec élimination de l'eau formée ;
b) la séparation des composés de départ non transformés de l'ester brut formé ;
c) le traitement de l'ester brut obtenu selon l'étape b) à la vapeur d'eau avec formation d'un flux secondaire volatil ; et
d) la séparation du flux secondaire volatil obtenu dans l'étape c),
**caractérisé en ce que** le flux secondaire volatil obtenu dans l'étape d) est séparé en une phase aqueuse et en une phase organique et la phase organique séparée est soumise à un traitement ultérieur à la vapeur d'eau et un flux de produits enrichi en polyolesters est obtenu comme résidu.

2. Procédé selon la revendication 1, **caractérisé en ce qu'**on réalise le traitement ultérieur à la vapeur d'eau à une température de 120 à 260°C, de préférence de 150 à 220°C, sur une période de 1 à 10 heures, de préférence de 1 à 5 heures.

3. Procédé selon la revendication 1 ou 2, **caractérisé en ce qu'**on réalise le traitement ultérieur à la vapeur d'eau par paliers avec des températures qui croissent d'un palier à l'autre.

4. Procédé selon l'une ou plusieurs des revendications 1 à 3, **caractérisé en ce que** le traitement ultérieur à la vapeur d'eau est réalisé en présence d'un adsorbant.

5. Procédé selon l'une ou plusieurs des revendications 1 à 4, **caractérisé en ce que** le traitement ultérieur à la vapeur d'eau est suivi d'un traitement au peroxyde d'hydrogène et, immédiatement après, d'un nouveau traitement à la vapeur d'eau.

6. Procédé selon l'une ou plusieurs des revendications 1 à 5, **caractérisé en ce qu'**on traite l'ester brut par des composés oxydants immédiatement avant l'étape c).

7. Procédé selon la revendication 6, **caractérisé en ce qu'**on utilise, comme composés oxydants, un composé peroxyde, l'ozone ou des gaz contenant de l'ozone.

8. Procédé selon la revendication 7, **caractérisé en ce qu'**on utilise, comme composé peroxyde, du peroxyde d'hydrogène.

9. Procédé selon l'une ou plusieurs des revendications 1 à 8, **caractérisé en ce qu'**on transforme, dans l'étape a), les polyols avec les acides monocarboxyliques en présence d'un catalyseur.

10. Procédé selon la revendication 9, **caractérisé en ce qu'**on utilise, comme catalyseur, le titane, le zirconium, l'hafnium, le fer, le zinc, le bore, l'aluminium ou l'étain en tant qu'éléments ou sous forme de leurs composés.

11. Procédé selon l'une ou plusieurs des revendications 1 à 10, **caractérisé en ce qu'**on transforme, dans l'étape a), les polyols avec les acides monocarboxyliques en présence d'un adsorbant.

12. Procédé selon la revendication 11, **caractérisé en ce qu'**on utilise, comme adsorbant, le silicagel, le gel silicique, le kieselguhr, l'oxyde d'aluminium, l'oxyde d'aluminium hydraté, des argiles, des carbonates ou le charbon activé.

13. Procédé selon l'une ou plusieurs des revendications 1 à 12, **caractérisé en ce qu'**on utilise, comme polyols, le di-triméthylolpropane, le di-pentaérythritol, le diéthylèneglycol, le triéthylèneglycol, le tétraéthylèneglycol, le dipropylèneglycol, le tripropylèneglycol ou le tétrapropylèneglycol.

14. Procédé selon l'une ou plusieurs des revendications 1 à 13 pour la préparation de di-2-éthylhexanoate de triéthylèneglycol, de di-n-heptanoate de tétraéthylèneglycol, de di-2-éthylbutyrate de triéthylèneglycol, de di-n-heptanoate de triéthylèneglycol ou de di-2-éthylhexanoate de tétraéthylèneglycol.
